# EUROPEAN PATENT APPLICATION

(11) **EP 3 103 411 A1**
(43) Date of publication of application: **14.12.2016**
(21) Application number: 15746625.1
(22) Date of filing: 28.01.2015
(51) Int. Cl.: A61B 90/00, A61B 18/12, A61B 1/00

(54) **SURGICAL SYSTEM AND METHOD FOR OPERATING SURGICAL SYSTEM**

(30) Priority: 07.02.2014 JP 2014022609
(71) Applicant: Olympus Corporation, Hachioji-shi Tokyo 192-8507 (JP)
(72) Inventor: YAMAMURA, Nahoko, Hachioji-shi, Tokyo 192-8507 (JP)
(74) Representative: Gunzelmann, Rainer
(86) International application number: PCT/JP2015/052328
(87) International publication number: WO 2015/119012

(57) **Abstract**

A surgical system (I) is provided with: a first medical instrument (2) that is disposed inside a body cavity and that has an observation unit (22) and a marking unit (23) that is capable of being positioned with respect to the body cavity; a second medical instrument (4) that is disposed outside the body cavity and that has a treatment section (42) and a driving unit (44) for driving the treatment section (42); a distance measuring unit (8) that measures the distance between the marking unit (23) and the treatment section (42); and a control unit (61) that controls the driving unit (44) on the basis of the distance measured by the distance measuring unit (8).

## Description

### {Technical Field}

The present invention relates to a surgical system and a surgical-system operating method.

### {Background Art}

In conventional laparoscopy endoscopy cooperative surgery (LECS), surgery is performed while observing a surgery area from both inside and outside a body cavity by using a laparoscope and an endoscope (for example, see PTL 1). For example, when a lesion existing on an inner wall of the stomach is removed, a removal line is determined by observing the lesion from inside the stomach by using an endoscope, and the stomach wall is incised along the determined removal line from outside the stomach by using a treatment tool, such as a surgical knife, thereby making it possible to minimize the removal area.

In this LECS, because a doctor determines the position of the removal line on the basis of the position of the endoscope in the body cavity, it is important to know the correct position of the endoscope in the body cavity. Thus, in PTL 1, a magnet or an LED is provided at a distal end of the endoscope, and a magnetic field from the magnet or light from the LED is detected outside the body cavity, thereby making it possible to detect the position of the endoscope in the body cavity.

### {Citation List}

### {Patent Literature}

{PTL 1} Japanese Unexamined Patent Application, Publication No. Hei 6-285042

### {Summary of Invention}

### {Technical Problem}

However, for example, the diameter of a polyp formed on the body-cavity inner wall ranges from about 20 mm to 50 mm, and thus, in order to minimize the removal area, it is required that a treatment tool be positioned with millimeter positional accuracy with respect to a determined removal line. Therefore, as in PTL 1, when a doctor manually positions the treatment tool on the basis of the detected position of the endoscope, the doctor is required to have an extremely high level of skill. In particular, in PTL 1, the doctor needs to identify the position of the endoscope in the body cavity with the help of sound output according to the intensity of the magnetic field or the brightness of the light emitted from the LED. In this way, a method of determining a position where the treatment tool should be disposed, on the basis of a sense of the doctor, has a problem in that a large burden is placed on the doctor, and accurate positioning of the treatment tool is difficult.

The present invention has been made in view of the above-described circumstances, and an object thereof is to provide a surgical system and a surgical-system operating method capable of, in LECS for giving treatment while observing a body cavity from both inside and outside, accurately positioning a treatment tool from outside with respect to a treatment position in the body cavity determined through observation from inside and accurately treating the determined treatment position.

### {Solution to Problem}

In order to achieve the above-described object, the present invention provides the following solutions.

According to a first aspect, the present invention provides a surgical system including: a first medical instrument that is disposed inside a body cavity and that has an observation unit for observing the body cavity and a marking unit capable of being positioned with respect to the body cavity; a second medical instrument that is disposed outside the body cavity and that has a treatment section for treating the body cavity and a driving unit for driving the treatment section; a distance measuring unit that measures the distance between the marking unit and the treatment section; and a control unit that controls the driving unit on the basis of the distance measured by the distance measuring unit.

According to the first aspect of the present invention, the control unit controls the driving unit on the basis of the distance between the marking unit of the first medical instrument, which is located inside the body cavity, and the treatment section of the second medical instrument, which is located outside the body cavity, thereby moving the treatment section. Therefore, the marking unit is positioned at the treatment position for a lesion etc. specified on the basis of an image of an inside of the body cavity observed by the observation unit, thereby making it possible to position the treatment section at an appropriate position with respect to the treatment position, with the body-cavity wall located therebetween.

In this way, positioning of the treatment section with respect to the treatment position is automatized, thereby enabling high-accuracy positioning of the treatment section. Accordingly, it is possible to accurately position the treatment tool from outside with respect to a treatment position in the body cavity that is determined through observation from inside and to accurately treating the determined treatment position.

In the above-described first aspect, the control unit may control the driving unit so as to position the treatment section at a position where the distance is equal to or less than a predetermined first threshold.

By doing so, the predetermined first threshold is set to a sufficiently small value (for example, a value approximately equal to the thickness dimension of the body-cavity wall), thereby making it possible to position the treatment section at a position substantially facing the marking unit, with the body-cavity wall located therebetween. Accordingly, it is possible to accurately recognize the position of the lesion in the body cavity from outside the body cavity.

In the above-described first aspect, the distance measuring unit may repeatedly measure the distance; and the control unit may cause the driving unit to repeatedly perform driving so as to reposition the treatment section at a position where the distance is equal to or less than the predetermined first threshold every time the distance measured by the distance measuring unit exceeds the predetermined first threshold.

By doing so, when the marking unit is moved, the control unit detects the movement as an increase in the distance measured by the distance measuring unit and controls the driving unit, thereby repositioning the treatment section at a position substantially facing the moved marking unit, with the body-cavity wall located therebetween. By making the treatment section track the movement of the marking unit in this way, the tissue can be treated along the trajectory of the marking unit.

In the above-described first aspect, the control unit may control the driving unit so as to position the treatment section at a position where the distance is greater than a predetermined second threshold.

By doing so, movement of the treatment section is permitted only outside a spherical area in which the marking unit is located at the center and whose radius is the predetermined second threshold. Accordingly, for example, in a procedure, such as lesion removal, in which treatment is given to an outside of the lesion, not to an inside of the lesion, it is possible to give treatment while conserving the lesion, by positioning the marking unit at the center of the lesion.

In the above-described first aspect, the control unit may have: a first mode that includes an automatic positioning mode for controlling the driving unit so as to position the treatment section at a position where the distance is equal to or less than the predetermined first threshold; a second mode that includes a tracking mode for causing the driving unit to repeatedly perform driving so as to reposition the treatment section at a position where the distance is equal to or less than the predetermined first threshold every time the distance measured by the distance measuring unit exceeds the predetermined first threshold; and a third mode that includes a marking-unit avoidance mode for controlling the driving unit so as to position the treatment section at a position where the distance is greater than a predetermined second threshold that is greater than the predetermined first threshold; and further comprises a mode selecting unit that causes an operator to alternatively select the first mode, the second mode, and the third mode.

By doing so, the operator can select the optimum mode for the specifics of the treatment, the situation, etc., by using the mode selecting unit.

According to a second aspect, the present invention provides a surgical-system operating method including: a distance measuring step of measuring the distance between a marking unit that is positioned inside a body cavity and a treatment section of a medical instrument that is positioned outside the body cavity; and a treatment-section moving step of moving the treatment section on the basis of the distance measured in the distance measuring step.

### {Advantageous Effects of Invention}

According to the present invention, an advantageous effect is afforded in that, in LECS for giving treatment while observing a body cavity from both inside and outside, it is possible to accurately position the treatment tool from outside with respect to a treatment position in the body cavity determined through observation from inside and to accurately treating the determined treatment position.

### {Brief Description of Drawings}

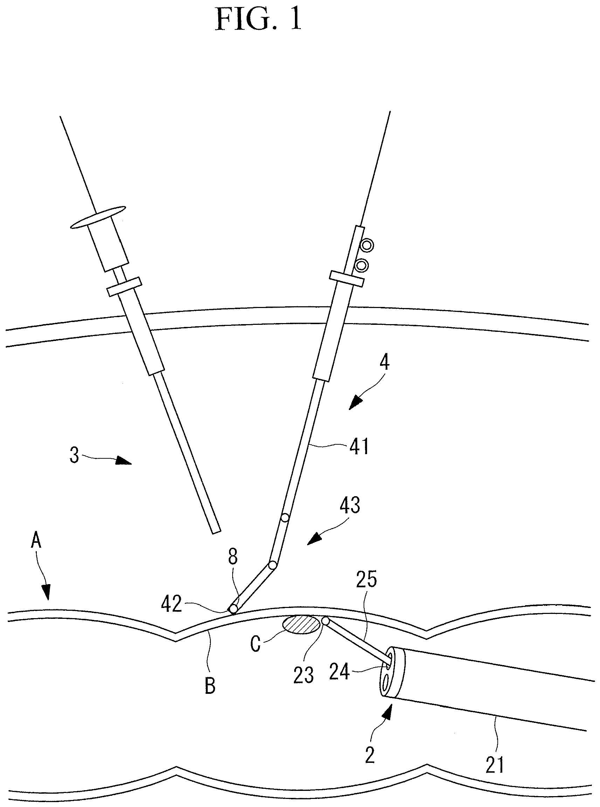

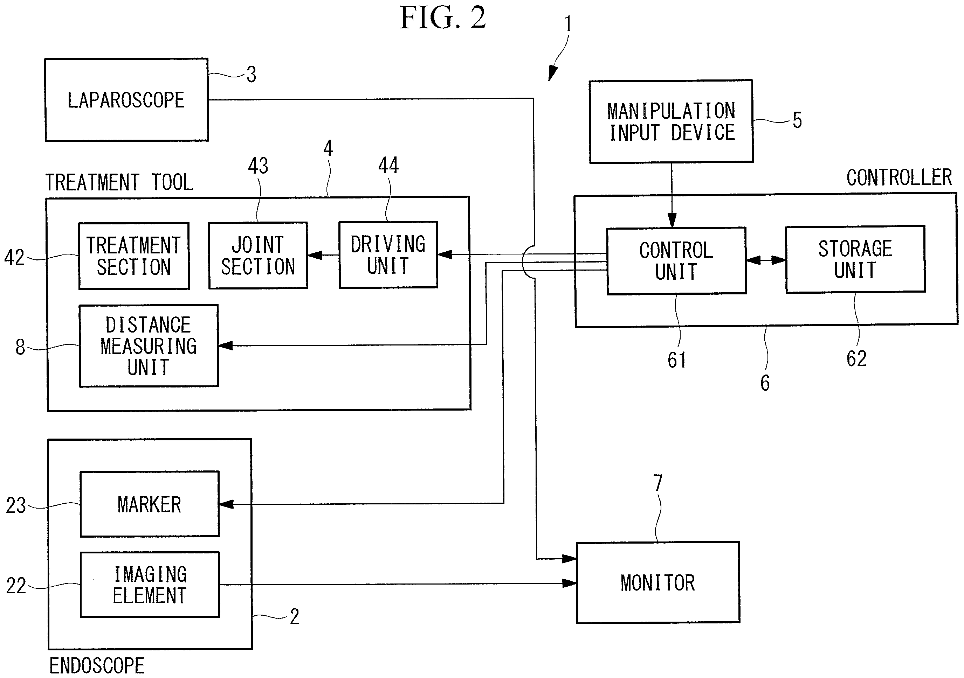

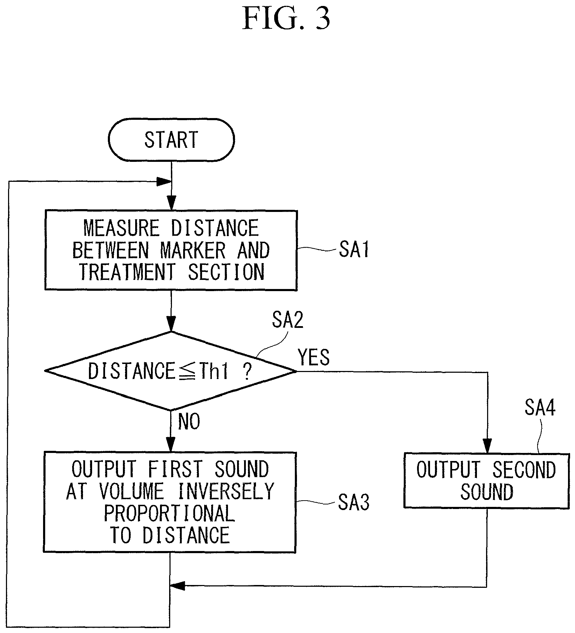

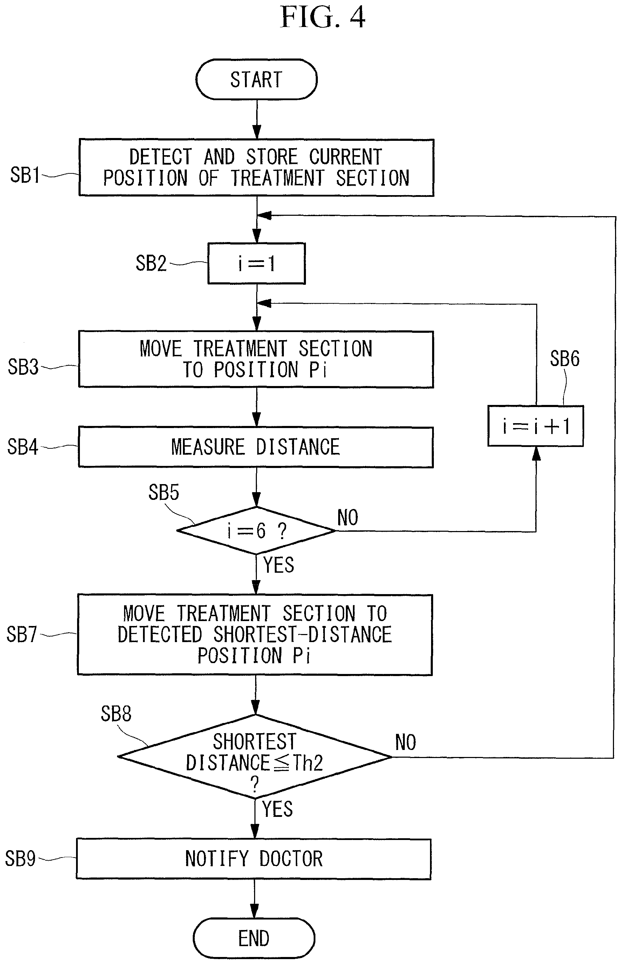

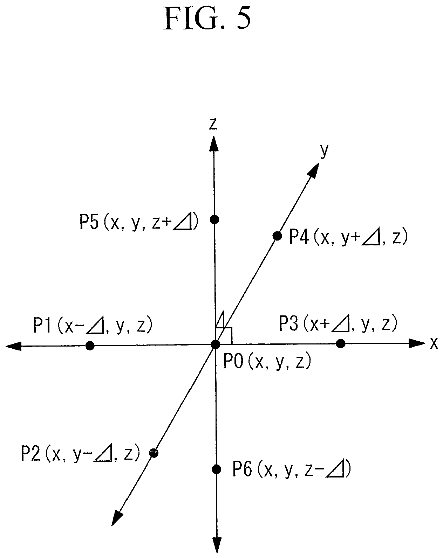

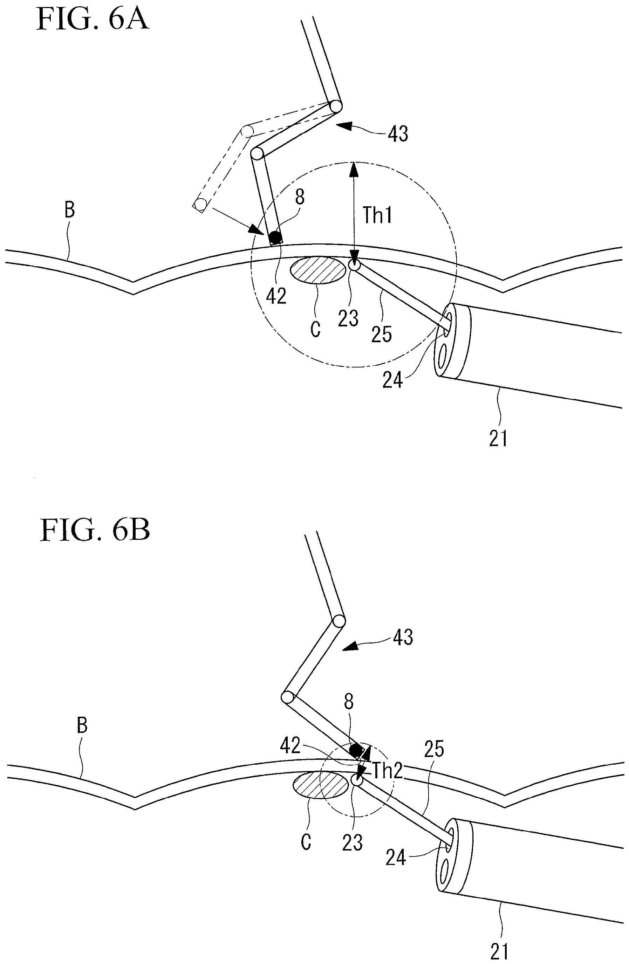

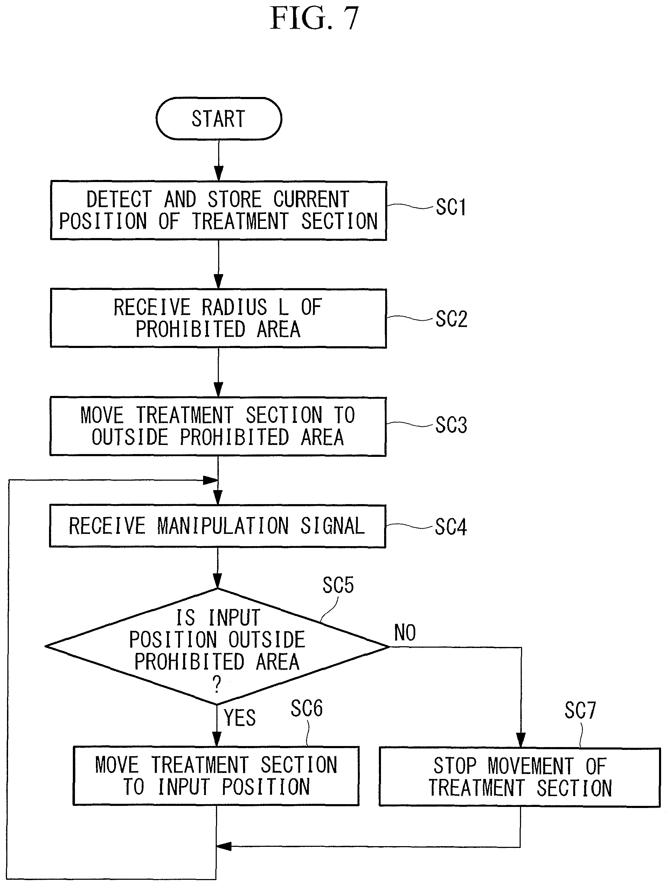

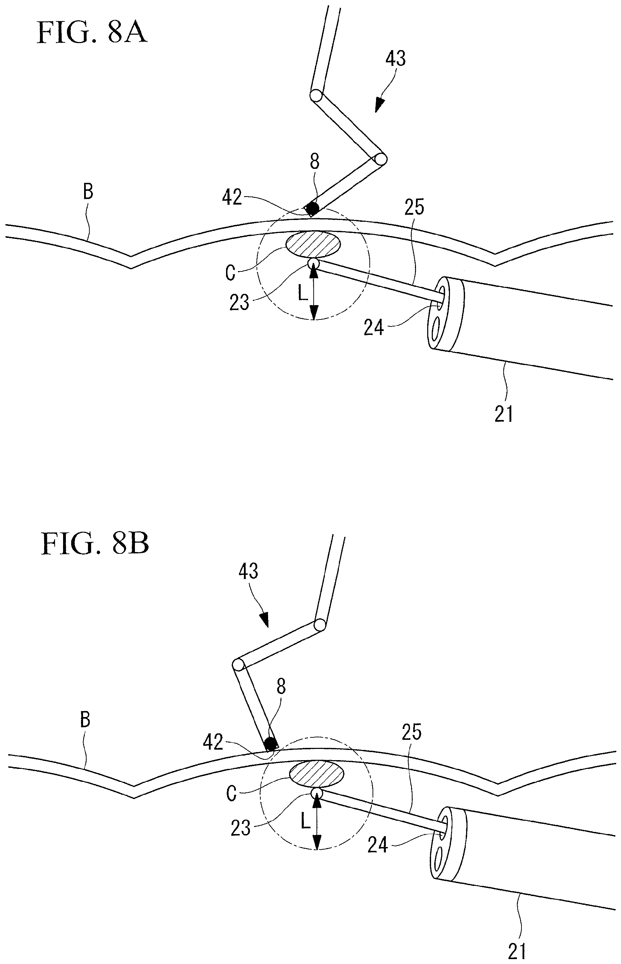

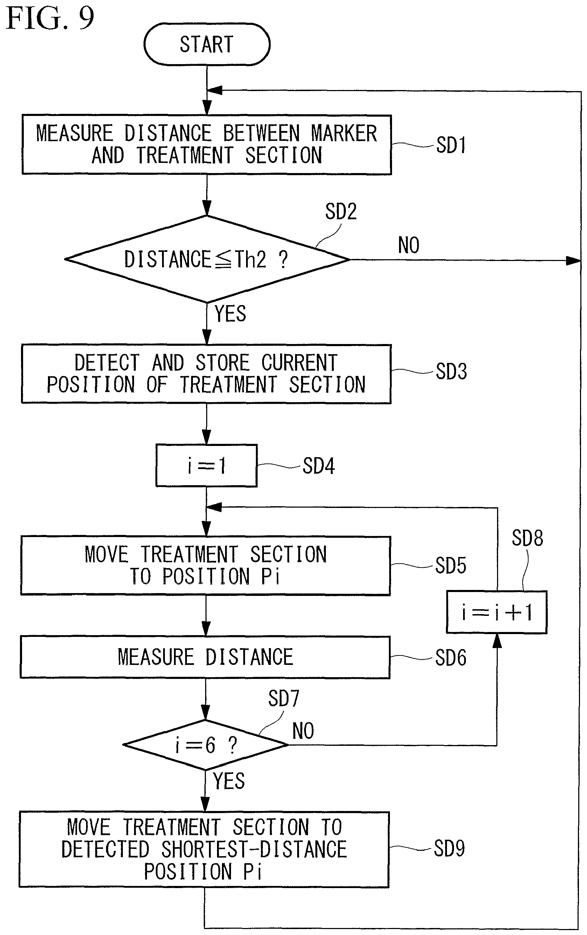

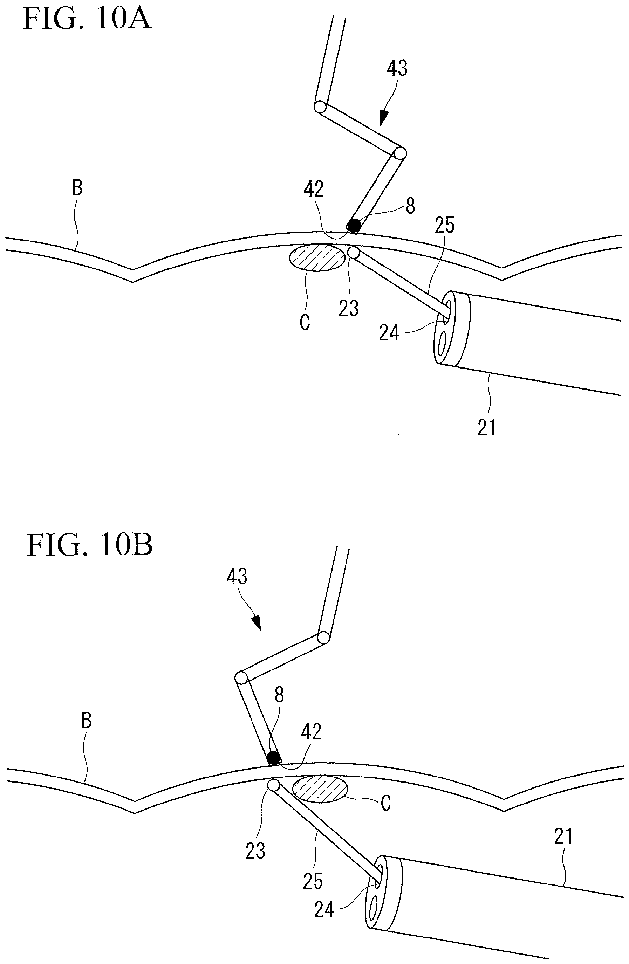

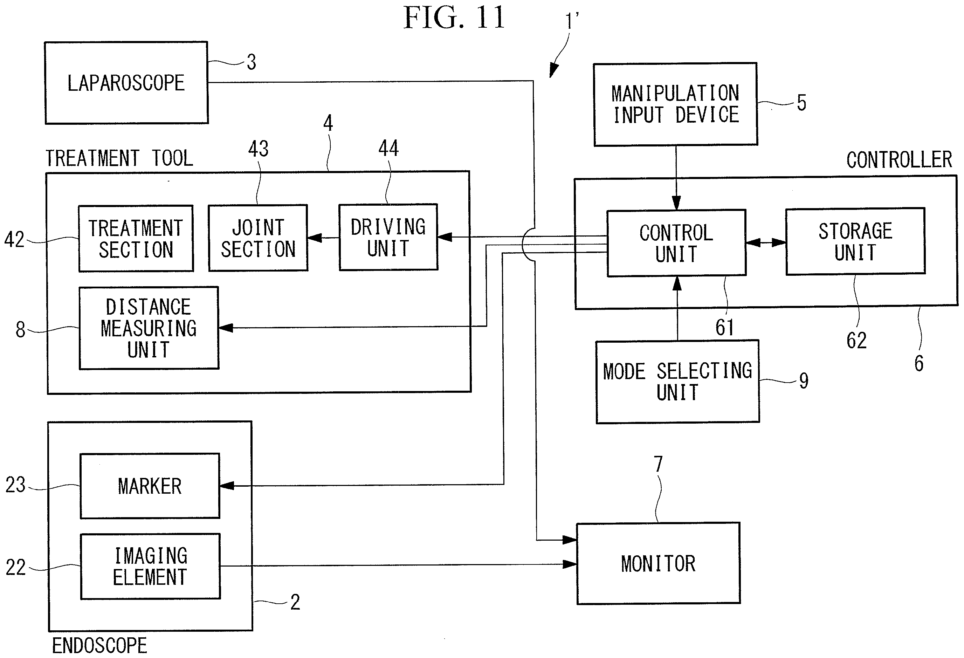

{Fig. 1} Fig. 1 is an overall configuration view showing the state in which a surgical system according to a first embodiment of the present invention is used.
{Fig. 2} Fig. 2 is a functional block diagram of the surgical system according to the first embodiment of the present invention.
{Fig. 3} Fig. 3 is a flowchart for explaining a "manual positioning mode".
{Fig. 4} Fig. 4 is a flowchart for explaining an "automatic positioning mode".
{Fig. 5} Fig. 5 is a view for explaining Steps SB2 to SB7 in the flowchart shown in Fig. 4.
{Fig. 6A} Fig. 6A is a view for explaining the movement of a treatment tool in the "automatic positioning mode".
{Fig. 6B} Fig. 6B is a view for explaining the movement of the treatment tool in the "automatic positioning mode".
{Fig. 7} Fig. 7 is a flowchart for explaining a "lesion avoidance mode" in a surgical system according to a second embodiment of the present invention.
{Fig. 8A} Fig. 8A is a view for explaining the movement of the treatment tool in the "lesion avoidance mode".
{Fig. 8B} Fig. 8B is a view for explaining the movement of the treatment tool in the "lesion avoidance mode".
{Fig. 9} Fig. 9 is a flowchart for explaining a "tracking mode" in a surgical system according to a third embodiment of the present invention.
{Fig. 10A} Fig. 10A is a view for explaining the movement of the treatment tool in the "tracking mode".
{Fig. 10B} Fig. 10B is a view for explaining the movement of the treatment tool in the "tracking mode".
{Fig. 11} Fig. 11 is a functional block diagram of a surgical system according to a fourth embodiment of the present invention.

### {Description of Embodiments}

### First Embodiment

A surgical system 1 according to a first embodiment of the present invention will be described below with reference to Figs. 1 to 6B.

The surgical system 1 of this embodiment is used for laparoscopy and endoscopy cooperative surgery (LECS) in which a body cavity A is treated from outside while observing the body cavity A from both inside and outside by using an endoscope 2 and a laparoscope 3, as shown in Fig. 1.

Specifically, as shown in Fig. 2, the surgical system 1 is provided with the endoscope 2, the laparoscope 3, a treatment tool 4, a manipulation input device 5 that is manipulated by a doctor (operator), and a controller 6 that controls the treatment tool 4 on the basis of an input to the manipulation input device 5.

The endoscope 2 is provided with an elongated flexible insertion section 21 that can be inserted into the body cavity A and an imaging element (observation unit) 22 that is built into the distal end of the insertion section 21, and a video of the inside of the body cavity A acquired by the imaging element 22 is sent to a monitor 7.

The endoscope 2 has a marker (marking unit) 23 that produces a signal (for example, magnetic field, light, heat, or voltage) that propagates from the inside of the body cavity A to the outside through a body-cavity wall B. The marker 23 is provided, for example, at the distal end of a wire 25 that is inserted, movably in the longitudinal direction, into a channel 24 formed through the insertion section 21 along the longitudinal direction. The doctor can move the marker 23 in the body cavity A by manipulating a base end portion of the wire 25 and can position the marker 23 with respect to an inner wall of the body cavity A by fixing the wire 25 with respect to the insertion section 21. Instead of the wire 25, a desired treatment tool that can be inserted into the channel 24 may be used.

The laparoscope 3 can be percutaneously inserted into the body and sends an acquired video of the inside of the body to the monitor 7.

The treatment tool 4 is provided with an elongated rigid body section 41 that can be percutaneously inserted into the body, a treatment section 42 that is provided close to a distal end of the body section 41 to treat tissue, a joint section 43 that couples the body section 41 with the treatment section 42, and a driving unit 44 that drives the joint section 43. In this embodiment, a description will be given of a case in which an electrocautery knife (hereinafter, also referred to as electrocautery knife 42) is provided as the treatment section 42; however, the treatment section 42 may be of another type, such as forceps or scissors.

The electrocautery knife 42 is provided with a distance measuring unit 8 that measures the distance between the electrocautery knife 42 and the marker 23 by detecting a signal produced by the marker 23.

Here, examples of the combination of the marker 23 and the distance measuring unit 8 include: a magnet (permanent magnet or electromagnet) and a Hall element or coil; a near-infrared laser source and a photodetector; a phototransmitter and a photoreceiver; a heating element and a thermal detector; and an AC-voltage generator and an impedance detector. In this way, the distance measuring unit 8 detects magnetism, the intensity of light, temperature, or the magnitude of impedance and measures the distance between the marker 23 and the electrocautery knife 42, which are disposed with the body-cavity wall B located therebetween, on the basis of the obtained detected value.

The joint section 43 swingably supports the electrocautery knife 42 in two-dimensional directions intersecting the longitudinal direction of the body section 41.

The driving unit 44 drives the joint section 43 on the basis of a control signal received from the controller 6, thereby moving the electrocautery knife 42 in two-dimensional directions intersecting the longitudinal direction of the body section 41.

The manipulation input device 5 generates a manipulation signal corresponding to a manipulation performed by the doctor and sends the generated manipulation signal to the controller 6.

The controller 6 is provided with a control unit 61 that controls the endoscope 2 and the treatment tool 4, and a storage unit 62.

The control unit 61 has a "manual positioning mode" in which manipulation of the treatment tool 4 via the manipulation input device 5 is permitted, and the driving unit 44 is controlled according to a manipulation input to the manipulation input device 5 by the doctor and an "automatic positioning mode" in which manipulation of the treatment tool 4 via the manipulation input device 5 is prohibited, and the driving unit 44 is controlled on the basis of the distance measured by the distance measuring unit 8. The two modes can be alternatively selected by the doctor by using a switch or the like provided on the controller 6.

Next, the "manual positioning mode" and the "automatic positioning mode" will be described in detail.

Fig. 3 is a flowchart for explaining how the control unit 61 performs control in the "manual positioning mode".

When the doctor selects the "manual positioning mode", the control unit 61 first makes the distance measuring unit 8 measure the distance between the marker 23 and the electrocautery knife 42 (Step SA1). Then, if the measured distance is greater than a predetermined first threshold Th1 (NO in Step SA2), the control unit 61 causes a first sound to be output from a speaker (not shown) at a volume that is inversely proportional to the distance (Step SA3). On the other hand, if the measured distance is equal to or less than the predetermined first threshold Th1 (YES in Step SA2), the control unit 61 causes a second sound to be output, the second sound being different in pitch, timbre, melody, or the like from the first sound (Step SA4). The control unit 61 repeats the above-described Steps SA1 to SA4 while the "manual positioning mode" is selected. Here, the first threshold Th1 is set less than the radius of a movable area of the electrocautery knife 42, in which the electrocautery knife 42 can be moved when the joint section 43 is driven.

In the "manual positioning mode", the doctor moves the electrocautery knife 42, which is disposed outside the body cavity A, in a direction in which the first sound is increased, thereby making it possible to bring the electrocautery knife 42 close to the marker 23, which is disposed inside the body cavity A. Then, through a change from the first sound to the second sound, the doctor can recognize that the electrocautery knife 42 has been disposed inside a spherical area in which the marker 23 is located at the center and whose radius is the first threshold Th1, as shown in Fig. 6A.

Fig. 4 is a flowchart for explaining how the control unit 61 performs control in the "automatic positioning mode".

When the doctor selects the "automatic positioning mode", the control unit 61 first stores the current position of the electrocautery knife 42 with respect to the body section 41 (Step SB1) and sets the current position as a reference position P0. Next, as shown in Fig. 5, the control unit 61 moves the electrocautery knife 42 to a plurality of (in this example, six) predetermined positions Pi (i = 1, 2, 3, ..., 6), with the reference position P0 serving as the reference (Step SB3) and makes the distance measuring unit 8 measure the distance at each of the positions (distance measuring step SB4). The measured distance is stored in the storage unit 62 in association with the corresponding position Pi.

After the distances at all of the positions Pi have been measured (Steps SB2, SB5, and SB6), the control unit 61 selects the position where the measured distance is the shortest among all the distances stored in the storage unit 62, as the closest position, and disposes the electrocautery knife 42 at the closest position again (treatment-section moving step SB7). Then, the closest position is set as a new reference position P0, and the above-described Steps SB1 to SB6 are repeated (NO in Step SB8).

If the shortest distance is equal to or less than a predetermined second threshold (predetermined first threshold) Th2 (YES in Step SB8), after the electrocautery knife 42 is disposed at the closest position (Step SB7), completion of the movement of the electrocautery knife 42 is notified to the doctor, and the "automatic positioning mode" is ended (Step SB9). Here, the second threshold Th2 is a value equal to or slightly greater than the thickness of the body-cavity wall B (for example, the thickness of the body-cavity wall B + several millimeters). Specifically, in the "automatic positioning mode", the electrocautery knife 42 is disposed substantially facing the marker 23, with the body-cavity wall B located therebetween, as shown in Fig. 6B, and is eventually positioned at a position where the distance to the marker 23 is the shortest.

The control unit 61 may automatically switch from the "manual positioning mode" to the "automatic positioning mode". Specifically, in the "manual positioning mode", when the distance measured by the distance measuring unit 8 is equal to or less than the predetermined first threshold Th1, the control unit 61 may forcibly quit the "manual positioning mode" and start the "automatic positioning mode".

Next, the operation of the thus-configured surgical system 1 will be described.

In order to remove a lesion C that exists in the inner wall of the body cavity A by using the surgical system 1 of this embodiment, first, the doctor percutaneously inserts the laparoscope 3 and the treatment tool 4 into the body, disposes the electrocautery knife 42 outside the body cavity A, and disposes the laparoscope 3 at a position where the electrocautery knife 42 can be observed. Furthermore, the doctor inserts the endoscope 2 into the body cavity A, determines an incision line surrounding the lesion C while observing the lesion C with an endoscope video, and positions the marker 23 on the incision line.

Next, the doctor starts the "manual positioning mode". While the electrocautery knife 42 is disposed at a position away from the marker 23, the small first sound is output. The doctor manipulates the manipulation input device 5 to move the electrocautery knife 42 in a direction in which the first sound is increased, and searches for an area where the first sound is changed to the second sound. Then, the electrocautery knife 42 is positioned at a position where the second sound is output. Accordingly, the electrocautery knife 42 is positioned roughly with respect to the marker 23.

Next, the doctor starts the "automatic positioning mode". After that, the control unit 61 drives the joint section 43, thereby positioning the electrocautery knife 42 at a position substantially facing the marker 23, with the body-cavity wall B located therebetween. The doctor incises the body-cavity wall B with the electrocautery knife 42 at the position where it is eventually positioned.

Then, the doctor repeatedly performs positioning of the electrocautery knife 42 in the two modes, i.e., the "manual positioning mode" and the "automatic positioning mode", while moving the marker 23 along the incision line, and performs incision of the body-cavity wall B. Accordingly, the body-cavity wall B is incised along the initially-determined incision line, thus making it possible to remove the lesion C.

In this way, according to this embodiment, a desired incision position is marked with the marker 23, and fine positioning of the electrocautery knife 42 with respect to the marker 23 is automatically controlled, thereby making it possible to position the electrocautery knife 42 with respect to the incision position with a high degree of accuracy. For example, it is possible to achieve millimeter positioning accuracy, which is required for incision of the small lesion C whose diameter is about 20 mm to 50 mm. Accordingly, there is an advantage that the doctor incises the body-cavity wall B accurately along an ideal incision line that is determined on the basis of the endoscope video, thus making it possible to minimize the extent of removal of the body-cavity wall B.

In this embodiment, it is possible to provide a contact detecting unit that detects contact between the electrocautery knife 42 and the tissue and to detect, in the "automatic positioning mode", contact between the electrocautery knife 42 and the tissue when the electrocautery knife 42 is moved to the position Pi. The tissue contact unit is formed of, for example, a conductive sensor that electrically detects contact between the electrocautery knife 42 and the tissue.

In this configuration, when contact between the electrocautery knife 42 and the tissue is detected, the control unit 61 stops distance measurement at the position Pi and goes onto measurement at the next position Pi+1, or the control unit 61 measures the distance at a position where the electrocautery knife 42 is not brought into contact with the tissue.

By doing so, the electrocautery knife 42 can be prevented from being brought into strong contact with the tissue.

In this embodiment, operation of the treatment section 42 (in this embodiment, supply of a high-frequency current to the electrocautery knife 42) may be permitted only when the distance measured by the distance measuring unit 8 is equal to or less than the second threshold Th2.

By doing so, incision of the body-cavity wall B is allowed only when the electrocautery knife 42 is positioned on the incision line, and, when the electrocautery knife 42 is not positioned on the incision line, the electrocautery knife 42 is not operated even if the doctor instructs operation of the electrocautery knife 42. Accordingly, the doctor can perform incision only at a position determined by himself or herself.

In this embodiment, the treatment tool 4 and a treatment tool that is inserted into the channel 24 of the endoscope 2 may form electrodes for the bipolar-type electrocautery knife, and supply of a high-frequency current to the electrodes may be permitted only when the distance between the marker 23, which is provided at the distal end of the treatment tool in the endoscope 2, and the treatment tool 4 is equal to or less than the second threshold Th2.

By doing so, the doctor can also perform incision only at a position determined by himself or herself.

### Second Embodiment

Next, a second embodiment of the present invention will be described with reference to Fig. 7 to Fig. 8B.

This embodiment differs from the first embodiment in that the control unit 61 has a "lesion avoidance mode (marking-unit avoidance mode)" in addition to the "manual positioning mode" and the "automatic positioning mode". Therefore, in this embodiment, the "lesion avoidance mode" will be mainly described, and a description of configurations common to those of the first embodiment will be omitted.

In this embodiment, the "manual positioning mode", the "automatic positioning mode", and the "lesion avoidance mode" can be alternatively selected by the doctor by using the switch or the like provided on the controller 6.

Fig. 7 is a flowchart for explaining how the control unit 61 performs control in the "lesion avoidance mode".

The "lesion avoidance mode" is used after positioning of the electrocautery knife 42 with respect to the marker 23 in the "automatic positioning mode" is completed. After the electrocautery knife 42 is positioned at a position substantially facing the marker 23 in the "automatic positioning mode", when the "automatic positioning mode" is switched to the "lesion avoidance mode", the control unit 61 first stores the current position of the treatment section 42 (Step SC1). The position to be stored at this time is usually the position substantially facing the marker 23, with the body-cavity wall B located therebetween, which is eventually determined in the "automatic positioning mode", as shown in Fig. 8A. Therefore, the control unit 61 can obtain the position of the marker 23 from the current position of the electrocautery knife 42 and the second threshold Th2.

Next, the control unit 61 prompts the doctor to input a radius (the predetermined second threshold) L through the manipulation input device 5 (Step SC2). The radius L is the radius of a prohibited area in which the electrocautery knife 42 is prohibited from being disposed. Next, the control unit 61 moves the electrocautery knife 42 to outside the prohibited area, which has the radius L from the position of the marker 23 located at the center, as shown in Fig. 8B (Step SC3). Then, the control unit 61 permits the doctor to manipulate the electrocautery knife 42 through the manipulation input device 5 (Step SC4).

However, the control unit 61 measures, through calculation, the distance between the movement destination of the electrocautery knife 42, which is input to the manipulation input device 5 by the doctor, and the marker 23 and compares the obtained distance with the radius L, thereby determining whether or not the movement destination is outside the prohibited area (distance measuring step SC5). Then, if the movement destination of the electrocautery knife 42 is outside the prohibited area (YES in Step SC5), the control unit 61 moves the electrocautery knife 42 according to the operator's input (treatment-section moving step SC6). On the other hand, if the movement destination of the electrocautery knife 42 is inside the prohibited area (NO in Step SC5), the control unit 61 denies this input, thus making the electrocautery knife 42 remain at the current position (Step SC7). At this time, the control unit 61 may notify the doctor that the movement destination of the electrocautery knife 42 is inside the prohibited area.

Next, the operation of the thus-configured surgical system will be described.

In this embodiment, the doctor positions the marker 23 at the center of the lesion C, as shown in Fig. 8A. Next, as described in the first embodiment, the doctor positions the electrocautery knife 42 at a position substantially facing the marker 23, with the body-cavity wall B located therebetween, in the "manual positioning mode" and the "automatic positioning mode". Next, the doctor starts the "lesion avoidance mode" and remotely manipulates the electrocautery knife 42 by using the manipulation input device 5. In the "lesion avoidance mode", the doctor can manipulate the electrocautery knife 42 only outside the prohibited area, which has the radius L from the center of the lesion C.

In this way, according to this embodiment, the electrocautery knife 42 is prohibited from being moved to the vicinity of the lesion C, and incision with the electrocautery knife 42 is permitted only in an area surrounding the lesion C. Specifically, this embodiment is used in a case in which the body-cavity wall B needs to be incised at a position where the lesion C is avoided. Accordingly, there is an advantage that the doctor can incise, while conserving the lesion C, the periphery of the lesion C to remove the lesion C.

In this embodiment, instead of stopping the movement of the electrocautery knife 42 when the movement destination of the electrocautery knife 42, which is input to the manipulation input device 5 by the doctor, is inside the prohibited area, it is also possible to prohibit supply of a high-frequency current to the electrocautery knife 42, while moving the electrocautery knife 42 according to this input.

By doing so, it is possible to dispose the electrocautery knife 42 in the vicinity of the lesion C, while conserving the lesion C, because the movement of the electrocautery knife 42 is not restricted, and to improve the degree of freedom of the manipulation of the electrocautery knife 42.

In this embodiment, switching from the "manual positioning mode" to the "automatic positioning mode" and from the "automatic positioning mode" to the "lesion avoidance mode" may be automatically performed by the control unit 61. Specifically, when the distance measured by the distance measuring unit 8 in the "automatic positioning mode" is equal to or less than the predetermined first threshold Th1, the control unit 61 may forcibly quit the "manual positioning mode", start the "automatic positioning mode", and start the "lesion avoidance mode" after the "automatic positioning mode" is ended.

### Third Embodiment

Next, a third embodiment of the present invention will be described with reference to Fig. 9 to Fig. 10B.

This embodiment differs from the first embodiment in that the control unit 61 has a "tracking mode" instead of the "automatic positioning mode". Therefore, in this embodiment, the "tracking mode" will be mainly described, and a description of configurations common to those of the first embodiment will be omitted.

In this embodiment, the "manual positioning mode" and the "tracking mode" can be alternatively selected by the doctor by using the switch or the like provided on the controller 6.

Fig. 9 is a flowchart for explaining how the control unit 61 performs control in the "tracking mode".

The "tracking mode" is used after positioning of the electrocautery knife 42 with respect to the marker 23 in the "manual positioning mode" is completed. After the electrocautery knife 42 is disposed inside an area that has the radius Th1 from the marker 23 located at the center, in the "manual positioning mode", when the "manual positioning mode" is switched to the "tracking mode", the control unit 61 makes the distance measuring unit 8 repeatedly measure the distance between the marker 23 and the electrocautery knife 42 (distance measuring step SD1). Then, if the measured distance is greater than the second threshold Th2 (NO in Step SD2), the control unit 61 positions the electrocautery knife 42 with respect to the marker 23 through a similar process to that in the "automatic positioning mode" such that the distance becomes equal to or less than the second threshold Th2 (YES in Step SD2) (Steps SD3 to SD9). In short, Steps SD3 to SD9 in the "tracking mode" are the same as Steps SB2 to SB8 in the "automatic positioning mode".

In this way, in the "tracking mode", when the marker 23 is moved after the control unit 61 has positioned the electrocautery knife 42 at a position substantially facing the marker 23, with the body-cavity wall B located therebetween, as shown in Fig. 10A, the control unit 61 detects this movement from an increase in the distance between the marker 23 and the electrocautery knife 42 and, as shown in Fig. 10B, again positions the electrocautery knife 42 at a position substantially facing the marker 23, with the body-cavity wall B located therebetween. Accordingly, the electrocautery knife 42 is made to track the movement of the marker 23.

Next, the operation of the thus-configured surgical system will be described.

In this embodiment, as in the first embodiment, the doctor positions the marker 23 on the incision line determined by himself or herself and roughly positions the electrocautery knife 42 with respect to the marker 23 in the "manual positioning mode". Next, the doctor starts the "tracking mode". Accordingly, the electrocautery knife 42 is positioned at a position substantially facing the marker 23, with the body-cavity wall B located therebetween.

After incising the body-cavity wall B with the electrocautery knife 42 at the position where it is positioned, when the doctor moves the marker 23 to another position on the incision line, the electrocautery knife 42 automatically tracks the movement of the marker 23 and is again positioned at a position substantially facing the marker 23, with the body-cavity wall B located therebetween. The doctor repeats movement of the marker 23 and incision with the electrocautery knife 42 at a movement destination until incision at all desired positions is completed.

In this way, according to this embodiment, the electrocautery knife 42 automatically tracks movement of the marker 23, and thus the electrocautery knife 42 is always positioned at a position substantially facing the marker 23, with the body-cavity wall B located therebetween. Therefore, there is an advantage that the doctor moves the marker 23 along the incision line determined by himself or herself, thereby making it possible to incise the body-cavity wall B accurately along the incision line.

Furthermore, although the endoscope 2, which is to be inserted into the body cavity A, must be flexible in many cases, because the flexible endoscope 2 is curved by a pressing force from the body-cavity wall B, it is difficult to incise the body-cavity wall B with a treatment tool that is introduced in the body cavity A through the channel 24. Thus, a removal line is specified by the endoscope 2, and the rigid treatment tool 4, which can transfer the force to the body-cavity wall B, is used, thereby making it possible to easily perform incision.

In this embodiment, as in the first embodiment, supply of a high-frequency current to the electrocautery knife 42 may be permitted only when the distance measured by the distance measuring unit 8 is equal to or less than the second threshold Th2. Furthermore, the treatment tool 4 and a treatment tool that is inserted into the channel 24 of the endoscope 2 may form electrodes for the bipolar-type electrocautery knife 42, and supply of a high-frequency current to the electrocautery knife 42 may be permitted only when the distance between the marker 23, which is provided at the distal end of the treatment tool in the endoscope 2, and the treatment tool 4 is equal to or less than the second threshold Th2.

### Fourth Embodiment

Next, a surgical system 1' according to a fourth embodiment of the present invention will be described with reference to Fig. 11.

This embodiment differs from the first to third embodiments in that the control unit 61 is configured so as to be able to alternatively select a combination from the three types of modes, which are described in the first to third embodiments.

Specifically, the control unit 61 has a "first mode", a "second mode", and a "third mode". The "first mode" includes the "manual positioning mode" and the "automatic positioning mode". The "second mode" includes the "manual positioning mode" and the "tracking mode". The "third mode" includes the "manual positioning mode", the "automatic positioning mode", and the "lesion avoidance mode".

In this embodiment, the surgical system 1' is provided with a mode selecting unit 9 that can alternatively select the first mode, the second mode, and the third mode through a manipulation performed by the doctor. The mode selecting unit 9 may be provided in the manipulation input device 5 or in the controller 6.

According to the thus-configured surgical system 1', there is an advantage that a more suitable mode can be selected according to the specifics of the treatment and the situation, thus making it possible to further assist the doctor's accurate treatment.

In this embodiment, it is merely necessary for the "first mode" to include at least the "automatic positioning mode", for the "second mode" to include at least the "tracking mode", and for the "third mode" to include at least the "lesion avoidance mode (marking-unit avoidance mode)". Furthermore, it is also possible to alternatively select two desired modes from the "first mode", the "second mode", and the "third mode".

In the above-described first to fourth embodiments, it is also possible to provide two or more distance measuring units 8 at different positions on the electrocautery knife 42.

By doing so, the relative positions of the electrocautery knife 42 and the marker 23 can be obtained through calculation from the distances measured by the respective distance measuring units 8. Accordingly, by presenting the obtained relative positions to the doctor, the doctor can more accurately recognize the position of the marker 23.

In particular, in the "tracking mode", a movement vector when the marker 23 is moved is obtained from displacement of the relative position thereof. Therefore, the control unit 61 moves the electrocautery knife 42 by the obtained movement vector, thereby allowing the electrocautery knife 42 to track the marker 23. Accordingly, the responsiveness of the electrocautery knife 42 with respect to the movement of the marker 23 can be improved.

### {Reference Signs List}

- 1, 1': surgical system
- 2: endoscope (first medical instrument)
- 21: insertion section
- 22: imaging element (observation unit)
- 23: marker (marking unit)
- 24: channel
- 25: wire
- 3: laparoscope
- 4: treatment tool (second medical instrument)
- 41: body section
- 42: electrocautery knife, treatment section
- 43: joint section
- 44: driving unit
- 5: manipulation input device
- 6: controller
- 61: control unit
- 62: storage unit
- 7: monitor
- 8: distance measuring unit
- 9: mode selecting unit
- A: body cavity
- B: body-cavity wall
- C: lesion
- SB4, SC5, SD1: distance measuring step
- SB7, SC6, SD9: treatment-section moving step

## Claims

1. A surgical system comprising:
a first medical instrument that is disposed inside a body cavity and that has an observation unit for observing the body cavity and a marking unit capable of being positioned with respect to the body cavity;
a second medical instrument that is disposed outside the body cavity and that has a treatment section for treating the body cavity and a driving unit for driving the treatment section;
a distance measuring unit that measures the distance between the marking unit and the treatment section; and
a control unit that controls the driving unit on the basis of the distance measured by the distance measuring unit.

2. A surgical system according to claim 1, wherein the
control unit controls the driving unit so as to position the treatment section at a position where the distance is equal to or less than a predetermined first threshold.

3. A surgical system according to claim 2,
wherein the distance measuring unit repeatedly measures the distance; and
the control unit causes the driving unit to repeatedly perform driving so as to reposition the treatment section at a position where the distance is equal to or less than the predetermined first threshold every time the distance measured by the distance measuring unit exceeds the predetermined first threshold.

4. A surgical system according to claim 1, wherein the control unit controls the driving unit so as to position the treatment section at a position where the distance is greater than a predetermined second threshold.

5. A surgical system according to claim 1,
wherein the control unit has: a first mode that includes an automatic positioning mode for controlling the driving unit so as to position the treatment section at a position where the distance is equal to or less than the predetermined first threshold; a second mode that includes a tracking mode for causing the driving unit to repeatedly perform driving so as to reposition the treatment section at a position where the distance is equal to or less than the predetermined first threshold every time the distance measured by the distance measuring unit exceeds the predetermined first threshold; and a third mode that includes a marking-unit avoidance mode for controlling the driving unit so as to position the treatment section at a position where the distance is greater than a predetermined second threshold that is greater than the predetermined first threshold; and
further comprises a mode selecting unit that causes an operator to alternatively select the first mode, the second mode, and the third mode.

6. A surgical-system operating method comprising:
a distance measuring step of measuring the distance between a marking unit that is positioned inside a body cavity and a treatment section of a medical instrument that is positioned outside the body cavity; and
a treatment-section moving step of moving the treatment section on the basis of the distance measured in the distance measuring step.
